# EUROPEAN PATENT APPLICATION

(11) **EP 3 466 914 A1**
(43) Date of publication of application: **10.04.2019**
(21) Application number: 17195093.4
(22) Date of filing: 06.10.2017
(51) Int. Cl.: C07C 37/20, C07C 37/68, C07C 37/70, C07C 37/72, C07C 37/74, C07C 37/80, C07C 39/16

(54) **METHOD OF FORMING A BISPHENOL**

(71) Applicant: SABIC Global Technologies B.V., 4612 PX Bergen op Zoom (NL)
(72) Inventor: Lakhete, Prashil Prakash, 562125 Bangalore (IN); Ramamurthy, Krishna Kumar, Hagadur Village, Bengaluru - 560066 (IN); Kankekar, Prasad, 562125 Bangalore (IN); Gahane, Bharti, 562125 Bangalore (IN); Deshpande, Hareesh Shamrao, 562125 Bangalore (IN)
(74) Representative: Sabic Intellectual Property Group

(57) **Abstract**

In an embodiment, a method of forming a bisphenol, comprises feeding a phenolic compound and an oxomethylene compound into a reactive separation column comprising a catalyst and reacting the phenolic compound and the oxomethylene compound to produce the bisphenol and water; wherein the feeding comprises feeding the oxomethylene compound in the reactive separation column in an amount of greater than or equal to 6 wt% based on a total weight of the phenolic compound and the oxomethylene compound; feeding a dehydrating agent into the reactive separation column and removing a water stream comprising the water and the dehydrating agent from a top end of the reactive separation column; and removing a bisphenol stream comprising the bisphenol from the reactive separation column.

## Description

### BACKGROUND

Bisphenol A is commercially produced by the condensation reaction of acetone and two equivalents of phenol in the presence of a catalyst such as an ion-exchange resin. Bisphenol A is a high production volume compound with a world-wide estimated annual production of over 2 million tons. The demand for this compound is primarily due to its use as a monomer in the production of many high commodity materials such as epoxy resins and polycarbonates.

Commercially, bisphenol A is produced in a molar excess of phenol, typically 11 moles of phenol (about 94.7 weight percent (wt%)) to 1 mole of acetone (about 5.3 wt%), allowing the phenol to act both as a solvent and as a reactant. This excess of phenol allows for an increased selectivity to bisphenol A, but reduces the formation of the bisphenol-A adduct in the crystallizer, ultimately resulting in loss of bisphenol A product. The lost bisphenol A product is removed from the crystallizer in the mother liquor, which can comprise about 10 wt% of bisphenol A. Although the bisphenol A from the mother liquor can be recycled back to the main bisphenol A reactor, its recovery is unfortunately limited due to the limited solubility of bisphenol A in phenol.

Furthermore, forming bisphenols under these conditions results in both an increased number of bisphenol A reactors and crystallization units required in order to meet the high capacity demands of bisphenol production facilities. For example, the excess phenol requires use of a low weighted hourly space velocity (WHSV) through the bisphenol reactors of only 0.75 to 1, increasing the number and/or the size of the reactors needed. Likewise, the excess phenol and the recycle mother liquor reduces the purity of the product stream from the bisphenol A reactor that is added to the crystallization section to a para,para-bisphenol A (p,p-BPA) purity of only about 73 wt%. This low purity requires at least two to three stages of crystallization utilizing large sized crystallization units in order to obtain a purity of p,p-BPA of greater than 99.8 wt%.

Clearly, improved methods of forming a bisphenol such as bisphenol A are needed.

### BRIEF SUMMARY

Disclosed herein is a method of forming a bisphenol.

In an embodiment, a method of forming a bisphenol, comprising: feeding a phenolic compound and an oxomethylene compound into a reactive separation column comprising a catalyst and reacting the phenolic compound and the oxomethylene compound to produce the bisphenol and water; wherein the feeding comprises feeding the oxomethylene compound in the reactive separation column in an amount of greater than or equal to 6 wt% based on a total weight of the phenolic compound and the oxomethylene compound; wherein the oxomethylene compound has a formula: R^{k1}-(C=O)-R^{k2}, wherein R^{k1} and R^{k2} are each independently hydrogen, a halide, or a substituted or unsubstituted C₁₋₁₈ aliphatic group, wherein R^{k1} and R^{k2} can together form a C₅-₁₈ cycloalkyl group or a C₅-₁₈ cycloalkylene group; feeding a dehydrating agent into the reactive separation column and removing a water stream comprising the water and the dehydrating agent from a top end of the reactive separation column; and removing a bisphenol stream comprising the bisphenol from the reactive separation column.

The above described and other features are exemplified by the following figures and detailed description.

### BRIEF DESCRIPTION OF THE DRAWINGS

The following Figures are exemplary embodiments, which are provided to illustrate the present method, wherein the like elements are numbered alike. The figures are not intended to limit devices made in accordance with the disclosure to the materials, conditions, or process parameters set forth herein.
FIG. 1 is an illustration of an embodiment of a method or producing a bisphenol in a reactive separation column;
FIG. 2 is an illustration of an embodiment of a reactive separation column with multiple reactive stages;
FIG. 3 is an illustration of an embodiment of reactive separation columns in series;
FIG. 4 is an illustration of an embodiment of a supported catalyst configuration in a reactive separation column;
FIG. 5 is an illustration of an embodiment of a supported catalyst configuration having open channel regions in a reactive separation column;
FIG. 6 is an illustration of an embodiment of a supported catalyst configuration having corrugated channel regions in a reactive separation column; and
FIG. 7 is an illustration of an embodiment of a section of a corrugated channel region.

### DETAILED DESCRIPTION

Commercially, bisphenols, such as bisphenol A are produced by reacting a phenolic compound and an oxomethylene compound using a molar excess of the phenolic compound, in amounts of greater than 94 wt%, as this excess allows for an increased selectivity of the bisphenol. This molar excess of phenol though ultimately results in a reduced product yield as well as an increased cost associated with the increased size of both the bisphenol reactors and crystallization units. It was surprisingly discovered that a bisphenol (such as bisphenol A) could be formed from a feed comprising an increased amount of the oxomethylene compound if a dehydrating agent was also fed into the reactive separation column. Specifically, the method of forming the bisphenol comprises feeding a phenolic compound and an oxomethylene compound into a reactive separation column comprising a catalyst and reacting the phenolic compound and the oxomethylene compound to produce the bisphenol and water; wherein the feeding comprises feeding the oxomethylene compound in the reactive separation column in an amount of greater than or equal to 6 wt% based on a total weight of the phenolic compound and the oxomethylene compound; feeding a dehydrating agent into the reactive separation column and removing a water stream comprising the water and the dehydrating agent from a top end of the reactive separation column; and removing a bisphenol stream comprising the bisphenol from the bottom of the reactive separation column.

The oxomethylene compound can have a formula: R^{kl}-(C=0)-Rk2, wherein R^{k1} and R^{k2} are each independently hydrogen, a halide, an alkyl halide, or a substituted or unsubstituted C₁₋₁₈ aliphatic group, wherein R^{k1} and R^{k2} can together form a C₅-₁₈ cycloalkyl group or a C₅-₁₈ cycloalkylene group. The oxomethylene compound can comprise at least one of acetone, acetophenone, hexafluoroacetone, butanone, benzophenone, acetaldehyde, formaldehyde, substituted or unsubstituted cyclohexanone, and 3,3,5-trimethylcyclohexanone. The oxomethylene compound can be present in an amount of 6 to 95 wt%, or 10 to 90 wt%, or 20 to 80 wt%, or 30 to 50 wt% based on a total weight of the phenolic compound and the oxomethylene compound fed into the reactive separation column. The oxomethylene compound can be present in an amount of greater than or equal to 6 wt%, or greater than or equal to 7 wt, or 7 to 95 wt%, based on a total weight of the phenolic compound and the oxomethylene compound fed into the reactive separation column.

The phenolic compound can comprise at least one of phenol, cresol, 2-isopropylphenol, and 2-phenylphenol. The phenolic compound can be present in an amount of 5 to 94 wt%, or 10 to 90 wt%, or 20 to 20 wt%, or 50 to 70 wt% based on a total weight of the phenolic compound and the oxomethylene compound fed into the reactive separation column. The phenolic compound can be present in an amount of 5 to 94 wt%, or 10 to 93 wt% based on a total weight of the phenolic compound and the oxomethylene compound fed into the reactive separation column.

When the method comprises forming bisphenol A, the phenolic compound can comprise phenol and the oxomethylene compound can comprise acetone.

The oxomethylene compound and the phenolic compound can each independently be fed into the reactive separation column at more than one feed locations, or 2 to 10 feed locations along a height of the reactive separation column.

The method can further comprise feeding a bulk promoter to the reactive separation column. The bulk promoter can comprise a mercaptan promoter, for example, at least one of methyl mercaptan (MM), ethyl mercaptan, 2,2-bis(methylthio)propane, mercaptocarboxylic acid, and 3- mercaptopropionic acid (3-MPA).

The feed to the reactive separation column can comprise less than or equal to 2 wt%, or 0 to 2 wt%, or 0 to 1 wt% of water based on the total weight of the feed. The feed to the reactive separation column can comprise methanol in an amount of 250 to 5,000 parts per million by weight (ppm), or 250 to 4,000 ppm, or 260 to 3,000 ppm, or 260 to 2,000 ppm based on the total weight of the oxomethylene compound.

The dehydrating agent can comprise at least one of the oxomethylene compound (for example, acetone) and an inert gas (for example, nitrogen and argon). The dehydrating agent can be added in one or both of the gas phase and the liquid phase. For example, when the dehydrating agent comprises an oxomethylene compound, the oxomethylene compound can be added in one or both of the gas phase and the liquid phase such that a total amount of the oxomethylene compound added to the reactive separation column can be greater than or equal to 10 wt%, greater than or equal to 70 wt%, or 75 to 90 wt% based on a total weight of the phenolic compound and the oxomethylene compound fed into the reactive separation column. In other words, the oxomethylene compound can also function as the dehydrating agent when an increased amount of oxomethylene compound is added to the reactive separation column.

When the dehydrating agent comprises a gas phase agent, the gas phase dehydrating agent can be fed into one or both of a bottom end of the reactive separation column and a side of the reactive separation column. The gas phase dehydrating agent can be added to the reactive separation column at a velocity of 0.01 to 0.6 meters per second (m/s), or 0.05 to 0.45 m/s. When the dehydrating agent comprises an inert gas, the inert gas can be added at a temperature of 45 to 80 degrees Celsius (°C), or 55 to 85°C.

The dehydrating agent can be fed at more than one dehydrating feed locations, or 2 to 10 dehydrating feed locations along the height of the reactive separation column.

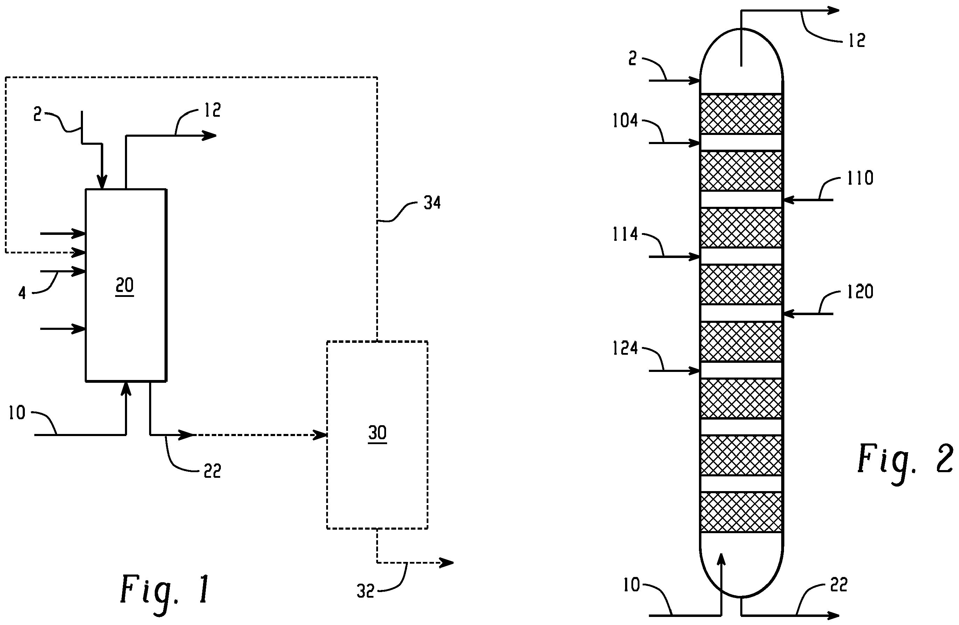

FIG. 1 illustrates an embodiment of a method of forming a bisphenol. Specifically, top feed stream 2 is added to the top end of reactive separation column 20; side feed streams 4 are added to the side of reactive separation column 20 along the height; and dehydrating feed stream 10 is added to a bottom end of reactive separation column 20. Depending on the components added, the method can comprise feeding two or more of each of these feed streams independently to the reactive separation column 20.

The method can comprise adding a liquid phase dehydrating agent such as acetone to reactive separation column 20 via one or more of top feed stream 2 and side feed stream 4. The liquid phase dehydrating agent can be added as a combined stream with the phenolic compound or they can be added as separate streams.

The method can comprise adding a gas phase dehydrating agent such as nitrogen to reactive separation column 20 via one or more of side feed stream 4 and dehydrating feed stream 10. The gas phase dehydrating agent can be added to reactive separation column 20 via dehydrating feed stream 10. The oxomethylene compound and the phenolic compound can be added as one or more combined streams and/or they can be added as separate streams. The oxomethylene compound and the phenolic compound can each independently be added via one or more of top feed stream 2 and side feed stream 4.

The method can comprise adding both a liquid phase dehydrating agent and a gas phase dehydrating agent. The liquid phase dehydrating agent can be added to reactive separation column 20 via one or more of top feed stream 2 and side feed stream 4 and the gas phase dehydrating agent can be added to reactive separation column 20 via one or more of side feed stream 4 and dehydrating feed stream 10.

FIG. 2 is an illustration of an embodiment of a reactive separation column. FIG. 2 illustrates that the oxomethylene compound can be added to the reactive separation column via oxomethylene compound feed streams 104, 114, and 124; a mixture of the oxomethylene compound and the phenolic compound can be added via top feed stream 2; and the dehydration agent can be added via dehydrating feed streams 10, 110, and 120. Water stream 12 comprising water, unreacted oxomethylene compound, unreacted phenolic compound, and the dehydration agent; and bisphenol stream 22 comprising the bisphenol, unreacted oxomethylene compound, unreacted phenolic compound, and impurities are removed from the reactive separation column.

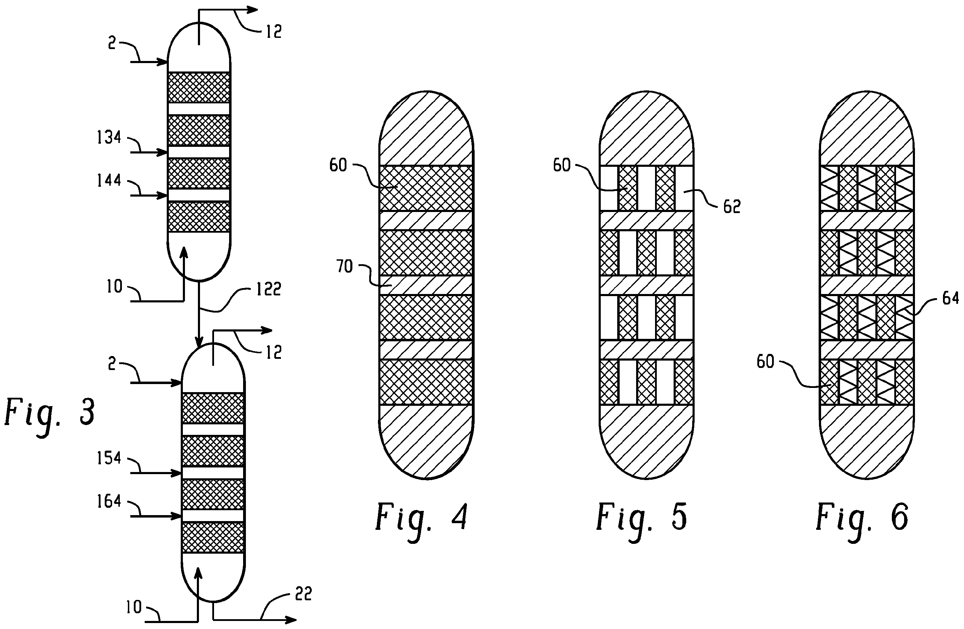

FIG. 3 is an illustration of an embodiment of a reactive separation column. FIG. 3 illustrates that the oxomethylene compound can be added to a first reactive separation column via oxomethylene compound feed streams 134 and 144; a mixture of the oxomethylene compound and the phenolic compound can be added via top feed stream 2; and the dehydration agent can be added via dehydrating feed stream 10. Water stream 12 comprising water, unreacted oxomethylene compound, unreacted phenolic compound, and the dehydration agent; and intermediate stream 122 comprising the bisphenol, unreacted oxomethylene compound, unreacted phenolic compound, and impurities are removed from the first reactive separation column. Intermediate stream 122 can be added to a second reactive distillation column in addition to oxomethylene compound feed streams 154 and 164, top feed stream 2, and dehydrating feed stream 10. Water stream 12 comprising water, unreacted oxomethylene compound, unreacted phenolic compound, and the dehydration agent; and bisphenol stream 22 comprising the bisphenol, unreacted oxomethylene compound, unreacted phenolic compound, and impurities are removed from the second reactive separation column.

As is understood by those of skill in the art, modifications to FIG. 2 and FIG. 3 can be made such as introducing a further reactive distillation column, using more or less catalyst stages, varying the concentration of the catalyst through the height of the column, adding more or less oxomethylene compound feed streams, adding more or less phenolic compound feed streams, adding more or less dehydrogenating feed streams, etc.

The reactive separation column can operate under isothermal reaction conditions at a temperature of 55 to 70°C, or 60 to 70°C. The reaction can occur at a weight hourly space velocity of 0.2 to 60 inverse hours (hr⁻¹), or 10 to 25 hr⁻¹, or 20 to 60 hr⁻¹. The pressure drop in the reactor can be 0.1 to 1.5 millibars per meter (mbar/m), or 0.1 to 0.75 mbar/m.

The reactive separation column can operate at a residence time from 2 to 90 min, or 2 to 60 min. It was found that as residence time increases, the selectivity for the bisphenol (such as p,p-BPA) decreases and an increased amount of impurity formation occurs. Therefore, it can be beneficial to operate the reactive separation column at a reduced residence time of less than or equal to 10 minutes, or 3 to 10 minutes, or 5 to 6 minutes to reduce the amount of impurity formation.

The method can be a single pass method such that 0 to 1 wt%, or 0 wt% of a bisphenol formed in the reactive separation column is added back into the column.

The method can comprise removing a water charged dehydrating agent stream and a bisphenol stream from the reactive separation column. The water charged dehydrating agent stream can be a water saturated dehydrating agent stream. For example, FIG. 1 illustrates water charged dehydrating agent stream 12 exiting from a top end of reactive separation column 20 and bisphenol stream 22 exiting from a bottom end of reactive separation column 20. The water charged dehydrating agent stream can comprise 0 to 10 wt% of water based on the total weight of the water charged dehydrating agent stream. The water charged dehydrating agent stream can comprise 75 to 100 wt% of the dehydrating agent based on the total weight of the water charged dehydrating agent stream.

The dehydrating agent can be separated from the water in the water charged dehydrating agent stream and at least a portion of the dehydrating agent can be recycled back into the reactive separation column. As the dehydrating agent can be removed from the reactive separation column as a gas, the recovered dehydrating agent can be separated from the water charged dehydrating agent stream and recycled back into the reactive separation column still in the gas phase, for example, via dehydrating feed stream 10.

The bisphenol can comprise at least one of bisphenol A (2,2-bis(4-hydroxyphenyl)propane), bisphenol AP (1,1-bis(4-hydroxyphenyl)-1-phenyl-ethane), bisphenol AF (2,2-bis(4-hydroxyphenyl)hexafluoropropane), bisphenol B (2,2-bis(4-hydroxyphenyl)butane), bisphenol BP (bis-(4-hydroxyphenyl) diphenylmethane), bisphenol C (2,2-bis(3-methyl-4-hydroxyphenyl)propane), bisphenol E (1,1-Bis(4-hydroxyphenyl)ethane), bisphenol F (bis(4-hydroxydiphenyl)methane), bisphenol G (2,2-bis(4-hydroxy-3-isopropyl-phenyl)propane), bisphenol PH (5,5'-(1-methylethyliden)-bis[1,1'-(bisphenyl)-2-ol]propane), bisphenol TMC (1,1-bis(4-hydroyphenyl)-3,3,5-trimethylcyclohexane), and bisphenol Z (1,1-bis(4-hydroxyphenyl)-cyclohexane). The bisphenol can comprise bisphenol A. The bisphenol stream can comprise 5 to 30 wt% of the bisphenol based on the total weight of the bisphenol stream.

The bisphenol can comprise para,para-bisphenol A and ortho,para-bisphenol A; and the para,para-bisphenol A can be present in an amount of greater than or equal to 80 wt%, or greater than or equal to 95 wt% based on the total weight of the para,para-bisphenol A and the ortho,para-bisphenol A.

The bisphenol stream can be directed to a separation column such that the bisphenol can be separated from unreacted reactants. At least a portion of the unreacted reacted reactants can be recycled back into the reactive separation column. For example, FIG. 1 illustrates that bisphenol stream 22 can be separated into separated bisphenol stream 32 comprising the bisphenol and unreacted recycle stream 34 comprising an unreacted phenolic compound and an unreacted oxomethylene compound in separation column 30 and that unreacted recycle stream 34 can be directed back into the reactive separation column 20.

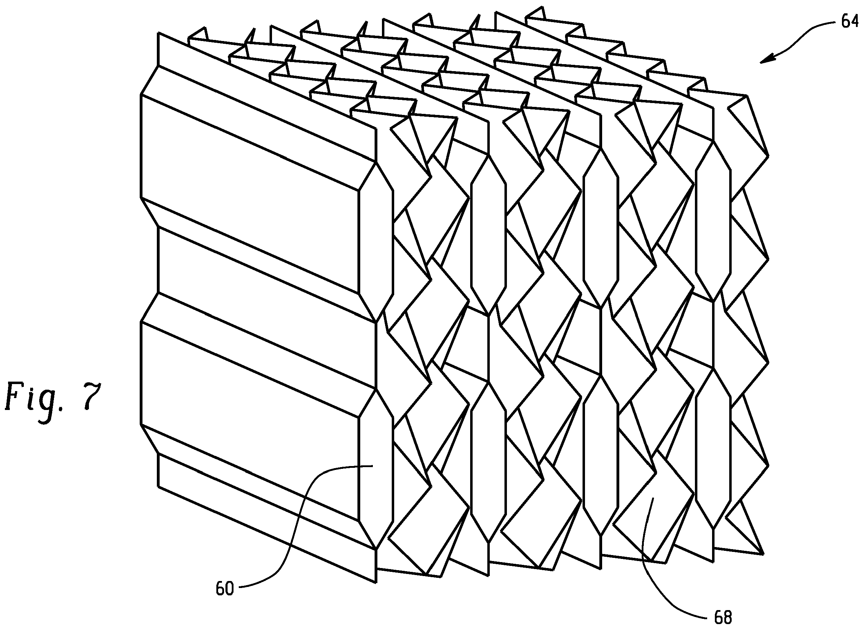

A catalyst can be present in the reactive separation column to facilitate the reaction between the oxomethylene compound and the phenolic compound. The catalyst can be distributed in multiple locations between the top and the bottom of the reactive separation column. The catalyst can be distributed in any suitable way within the column. For example, the catalyst can be evenly distributed throughout the column, such as in the form of structures packing including the catalyst. The catalyst can be distributed on several different trays in the column. The supported catalyst regions can be separated by a corrugated metal sheet having a plurality of through-hole, a wire mesh, or a metal foam. The catalyst can be unevenly distributed in the column, for example, as illustrated in FIG. 4, FIG. 5, and FIG. 6. FIG. 4 illustrates that the reactive separation column can comprise supported catalyst regions 60 separated by catalyst free regions 70. FIG. 5 illustrates that supported catalyst regions 60 can comprise open channel regions 62 that are free of the supported catalyst. FIG. 6 illustrates that supported catalyst regions 60 can comprise corrugated channel regions 64 that are free of the supported catalyst, but that provide an impeded flow path for the passing fluids. FIG. 7 is an illustration of a corrugated channel region 64. FIG. 7 illustrates that the supported catalyst region 60 can be located in a catalyst pocket and the corrugated channel region can comprise a plurality of open channels that are not linear in path.

The catalyst can comprise an ion-exchange resin (IER), such as a cation exchange resin. The catalyst can comprise at least one of a sulfonated, cross-linked polystyrene ion exchange, a phenol-formaldehyde sulfonic acid resin, and a formaldehyde sulfonic acid resin.

The sulfonated, cross-linked polystyrene ion exchange resin can be crosslinked with 2 to 4 wt% of divinylbenzene as the cross-linking agent (based on the total weight of initial monomers used during polymerization of the resin). The sulfonated, cross-linked polystyrene ion exchange resin can be either a mono- or a polydispersed material and can have a particle size of 300 to 1,200 micrometers. The sulfonated, cross-linked polystyrene ion exchange resin can comprise 0.5 to 20 mole percent (mol%), or 2 to 4 mol% of a repeat unit derived from a cross-linker relative to a total amount of repeat units.

The sulfonated, cross-linked polystyrene ion exchange resin can be sulfonated by introducing the cross-linked resin to a sulfonating reagent. The sulfonating reagent can comprise at least one of chlorosulfonic acid, sulfur trioxide, and sulfuric acid. The sulfonated, cross-linked polystyrene ion exchange resin can have an initial acid content of 0.01 to 0.2 milli-equivalents of acid groups per centimeters cubed of the catalyst.

The catalyst, for example, the sulfonated, cross-linked polystyrene ion exchange resin can comprise an attached promoter. The sulfonated, crosslinked resin can then be functionalized such that the resultant catalyst system comprises 5 to 35 mol%, or 10 to 30 mol%, 10 to 25 mol%, or 15 to 25 mol% of an attached promoter molecule based on the total moles of the sulfonic acid sites in the catalyst system. The attached promoter can comprise at least one thiol group, or at least two thiol groups, for example, 2 to 6, or 2 to 4 thiol groups.

The attached promoter can have the formula: 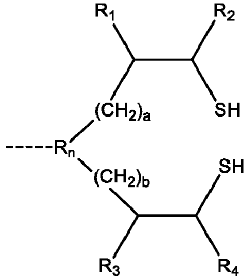 wherein a and b are each independently an integer of 0 to 5, Rₙ can be an amine group, a pyridine group, a phosphonium group, or a C₁₋₄ group (for example, a C₁₋₄ alkyl group); and R₁, R₂, R₃, and R₄ can each independently be -H, -OH, -SH, or an alkyl group (for example, a C₁₋₄ group).

The catalyst can have an average particle size of the catalyst is 300 to 1,500 micrometers, or 300 to 1,000 micrometers, or 350 to 500 micrometers at a moisture content of 60 to 90 wt%. For down-flow operation, the catalyst can have an average particle size of 600 to 1,500 micrometers at a moisture content of 60 to 90 wt%.

The catalyst can comprise catalyst particles located on a support. The support can have a void space of greater than or equal to 50 vol%, or 50 to 99 vol%, or 60 to 80 vol% based on a total volume of the support. The support can comprise a porous carrier having 5 to 200 pores per 2.54 centimeters. The support can have a pore volume of at least 90 volume percent (vol%) based on the total volume of the porous carrier. The number of pores per unit length can be determined in accordance with ASTM D3576-04 (2010), Procedure A. The pore volume can be determined using the following equation where the pore volume is equal to 1 - (foam density/material density). For example, for a foam having a foam density of 40 kilograms per meter cubed (kg/m³) that is made from a foam material of amorphous carbon having a density of 2,000 kg/m³, the pore volume is 0.98 (1-(40/2,000)).

The support can comprise at least one of a foam and a wire mesh. The support material can comprise at least one of carbon and a metal (for example, aluminum, chromium, iron, copper, silicon (for example, silicon carbide), and nickel). The metal can comprise stainless steel. Examples of porous carriers include those that are commercially available from ERG Aerospace Corporation, Oakland CA and from Alantum Corporation, Gyonggi-Do, Korea.

If desired, the separated bisphenol stream can be post-treated to further purify the bisphenol. The post-treatment can comprise crystallization of bisphenol to form crystals comprising a crystallized bisphenol and/or a crystallized bisphenol adduct. The crystallization can comprise a vacuum cooling step. The crystallization can be facilitated by adding water, for example, in an amount of less than or equal to 3 wt%, or 0.1 to 3 wt% based on the total weight of the product mixture. The crystals can be optionally separated, for example, by filtration and melted in a melting unit. If the melt comprises sulfur, then a base (for example, sodium hydroxide and potassium hydroxide) can be added to the melt to form a melt stream with a reduced sulfur content. The melted stream can be filtered, further purified, and then solidified, for example, in a flaking unit.

The bisphenols produced by this process can be used to manufacture a polycarbonate. A "polycarbonate" as used herein means compositions having repeating structural carbonate units of formula (1) 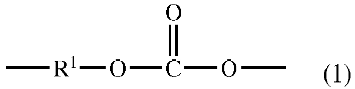 in which the R¹ groups contain aliphatic, alicyclic, and/or aromatic moieties (e.g., greater than or equal to 30%, or greater than or equal to 60%, of the total number of R¹ groups can contain aromatic moieties and the balance thereof are aliphatic or alicyclic). Optionally, each R¹ can be a C₆₋₃₀ aromatic group, that is, can contain at least one aromatic moiety. R¹ can be derived from the bisphenol.

The following examples are provided to illustrate the present disclosure. The examples are merely illustrative and are not intended to limit devices made in accordance with the disclosure to the materials, conditions, or process parameters set forth therein.

### Examples

In the Examples, selectivity for p,p-BPA was determined by dividing the amount of p,p-BPA produced by the reaction product in the effluent, both in wt%, times 100.

### Examples 1-5: Effect of acetone concentration in the feed

In Examples 1-5, a reactive separation column was configured in accordance with FIG. 1 except that there were no side feeds to the column. The reactive separation column had a 50 millimeter (mm) inner diameter and a height of 40 centimeters (cm). 72 grams (g) of a sulfonated polystyrene catalyst having 1,000 parts per million by weight of -SH groups embedded in a wire mesh was added to each stage of the reactor. The wire mesh had a free volume of 70 vol% based on the total volume of the wire mesh. In the examples, the amount of acetone was varied and the resultant amount of p,p-BPA analyzed. The results are described below and shown in Table 1.

In Example 1, top feed stream 2 comprising 7.16 wt% acetone, 92.28 wt% phenol, 0.28 wt% water, and 0.28 wt% 3-MPA was added to reactive separation column 20 at a flow rate of 16 kilograms per hour (kg/hr). The reactant mass was passed through the column 11 times to simulate an eleven stage reactor such that a total residence time in the reactor was 2.97 minutes. Nitrogen was added via dehydrating feed stream 10 at a flow rate of 1.5 kg/hr. The reaction was performed under isothermal conditions at a temperature of 65°C, a weight hourly space velocity of 20.2 hr⁻¹, and a residence time of 2.97 minutes in the column.

Bisphenol stream 22 was analyzed and found to comprise 2.73 wt% of acetone, 0.30 wt% of water, 11.75 wt% of p,p-BPA, and 0.38 wt% of o,p-BPA resulting in a p,p-BPA to o,p-BPA production weight ratio of 30.9 and an overall selectivity towards p,p-BPA of 98.9 wt% based on the total amount of BPA formed. The p,p-BPA production rate was determined to be 2.4 kilograms of p,p-BPA per kilogram of catalyst per hour (kg p,p-BPA/kg catalyst/hr). Considering the amount of water added in top feed stream 2 and removed in bisphenol stream 22 and assuming the theoretical amount of water produced based on the bisphenol product, it was determined that more than 98 wt% of the water formed during the reaction was removed via water charged dehydrating agent stream 12.

Comparing Example 1 to conventional methods of producing BPA that generally use more than 15 kilograms of catalyst to produce the same amount of BPA, illustrates that the present method can result in an almost 90 wt% decrease in the amount of catalyst needed.

| Table 1 | | | | | |
|---|---|---|---|---|---|
| Example | 1 | 2 | 3 | 4 | 5 |
| Acetone in stream 2 (wt%) | 7 | 13 | 33 | 79.7 | 79.7 |
| Nitrogen flow rate (kg/hr) | 1.5 | 0.8 | 0.4 | 0.2 | 0 |
| p,p-BPA to o,p-BPA wt ratio | 30.9 | 23.4 | 51.0 | 11.0 | 14.7 |
| p,p-BPA selectivity (%) | 98.9 | 99.0 | 92.0 | 82.0 | 82.4 |
| p,p-BPA production rate (kg p,p-BPA/kg catalyst/hr) | 2.4 | 1.9 | 5.1 | 1.9 | 1.9 |
| Water removed in stream 12 (wt%) | 98.5 | 93.7 | 100 | 74.0 | 70.0 |

In Example 2, top feed stream 2 comprising 13.33 wt% acetone, 85.95 wt% phenol, 0.27 wt% water, and 0.45 wt% 3-MPA was added to reactive separation column 20 at a flow rate of 16 kg/hr. The reactant mass was passed through the column 10 times to simulate a ten stage reactor. Nitrogen was added via dehydrating feed stream 10 at a flow rate of 0.8 kg/hr. The reaction was performed under isothermal conditions at a temperature of 60°C, a weight hourly space velocity of 22 hr⁻¹, and a residence time of 2.7 minutes in the column.

Bisphenol stream 22 was analyzed and found to comprise 7.10 wt% of acetone, 0.35 wt% of water, 14.90 wt% of p,p-BPA, and 0.64 wt% of o,p-BPA resulting in a p,p-BPA to o,p-BPA production weight ratio of 23.4 and an overall selectivity towards p,p-BPA of 99.0% based on the total amount of BPA formed. The p,p-BPA production rate was determined to be 1.9 kg p,p-BPA/kg catalyst/hr. Considering the amount of water added in top feed stream 2 and removed in bisphenol stream 22 and assuming the theoretical amount of water produced based on the bisphenol product, it was determined that more than 93 wt% of the water formed during the reaction was removed via water charged dehydrating agent stream 12, also referred to as water stream 12.

In Example 3, top feed stream 2 comprising 32.70 wt% acetone, 66.70 wt% phenol, 0.27 wt% water, and 0.33 wt% 3-MPA was added to reactive separation column 20 at a flow rate of 16 kg/hr. The reactant mass was passed through the column 2 times to simulate a two stage reactor. Nitrogen was added via dehydrating feed stream 10 at a flow rate of 0.4 kg/hr. The reaction was performed under isothermal conditions at a temperature of 55°C, a weight hourly space velocity of 55.5 hr⁻¹, and a residence time of 1.08 minutes in the column.

Bisphenol stream 22 was analyzed and found to comprise 0.25 wt% of water, 4.66 wt% of p,p-BPA, and 0.09 wt% of o,p-BPA resulting in a p,p-BPA to o,p-BPA production weight ratio of 51.0 and an overall selectivity towards p,p-BPA of 92.0% based on the total amount of BPA formed. The p,p-BPA production rate was determined to be 5.1 kg p,p-BPA/kg catalyst/hr. Considering the amount of water added in top feed stream 2 and removed in bisphenol stream 22 and assuming the theoretical amount of water produced based on the bisphenol product, it was determined that almost 100 wt% of the water formed during the reaction was removed via water stream 12.

Comparing Example 2 and Example 3, it is observed that increasing the flow rate of the acetone allowed for a reduced amount of nitrogen needed to remove nearly 100% of the water from the column.

In Example 4, top feed stream 2 comprising 79.70 wt% acetone, 19.70 wt% phenol, 0.27 wt% water, and 0.33 wt% 3-MPA was added to reactive separation column 20 at a flow rate of 7 kg/hr. The reactant mass was passed through the column 4 times to simulate a four stage reactor. Nitrogen was added via dehydrating feed stream 10 at a flow rate of 0.2 kg/hr. The reaction was performed under isothermal conditions at a temperature of 55°C, a weight hourly space velocity of 24.3 hr⁻¹, and a residence time of 2.46 minutes in the column.

Bisphenol stream 22 was analyzed and found to comprise 0.35 wt% of water, 9.56 wt% of p,p-BPA, and 0.86 wt% of o,p-BPA resulting in a p,p-BPA to o,p-BPA production weight ratio of 11.0 and an overall selectivity towards p,p-BPA of 82.0% based on the total amount of BPA formed. The p,p-BPA production rate was determined to be 1.9 kg p,p-BPA/kg catalyst/hr. Considering the amount of water added in top feed stream 2 and removed in bisphenol stream 22 and assuming the theoretical amount of water produced based on the bisphenol product, it was determined that 74 wt% of the water formed during the reaction was removed via water stream 12.

In Example 5, top feed stream 2 comprising 79.7 wt% acetone, 19.7 wt% phenol, 0.27 wt% water, and 0.33 wt% 3-MPA was added to reactive separation column 20 at a flow rate of 7 kg/hr. The reactant mass was passed through the column 4 times to simulate a four stage reactor. Nitrogen was not added to the column. The reaction was performed under isothermal conditions at a temperature of 55°C, a weight hourly space velocity of 24.3 hr⁻¹, and a residence time of 2.46 minutes in the column.

Bisphenol stream 22 was analyzed and found to comprise 0.31 wt% of water, 7.91 wt% of p,p-BPA, and 0.54 wt% of o,p-BPA resulting in a p,p-BPA to o,p-BPA production weight ratio of 14.7 and an overall selectivity towards p,p-BPA of 82.4% based on the total amount of BPA formed. The p,p-BPA production rate was determined to be 1.9 kg p,p-BPA/kg catalyst/hr. Considering the amount of water added in top feed stream 2 and removed in bisphenol stream 22 and assuming the theoretical amount of water produced based on the bisphenol product, it was determined that 70.0 wt% of the water formed during the reaction was removed via water stream 12.

Example 5 illustrates that the increased amount of acetone added results in a portion of the acetone functioning as the dehydrating agent, such that a portion of the acetone vaporizes in the column and removes the water. Comparing this example to Example 4, where the same amount of reactants were added to the reactive separation column 20 along with a nitrogen, it can be seen that similar amounts of p,p-BPA were produced.

Set forth below are non-limiting embodiments of the present disclosure.

Embodiment 1: A method of forming a bisphenol, comprising: feeding a phenolic compound and an oxomethylene compound into a reactive separation column comprising a catalyst and reacting the phenolic compound and the oxomethylene compound to produce the bisphenol and water; wherein the feeding comprises feeding the oxomethylene compound in the reactive separation column in an amount of greater than or equal to 6 wt% based on a total weight of the phenolic compound and the oxomethylene compound; wherein the oxomethylene compound has a formula: R^{k1}-(C=O)-R^{k2}, wherein R^{k1} and R^{k2} are each independently hydrogen, a halide, or a substituted or unsubstituted C₁₋₁₈ aliphatic group, wherein R^{k1} and R^{k2} can together form a C₅-₁₈ cycloalkyl group or a C₅-₁₈ cycloalkylene group; feeding a dehydrating agent into the reactive separation column and removing a water stream comprising the water and the dehydrating agent from a top end of the reactive separation column; and removing a bisphenol stream comprising the bisphenol from the reactive separation column.

Embodiment 2: The method of Embodiment 1, wherein the oxomethylene compound is present in an amount of 6 to 95%, or 10 to 90 wt%, or 20 to 80 wt%, or 30 to 50 wt% based on a total weight of the phenolic compound and the oxomethylene compound fed into the reactive separation column. The oxomethylene compound can be present in an amount of greater than or equal to 6 wt%, or greater than or equal to 7 wt, or 7 to 95 wt% based on a total weight of the phenolic compound and the oxomethylene compound fed into the reactive separation column. The oxomethylene compound can be present in an amount of 75 to 95 wt% based on a total weight of the phenolic compound and the oxomethylene compound fed into the reactive separation column.

Embodiment 3: The method of any one of the preceding embodiments, wherein the oxomethylene compound comprises at least one of acetone, acetophenone, hexafluoroacetone, butanone, benzophenone, acetaldehyde, formaldehyde, substituted or unsubstituted cyclohexanone, and 3,3,5- trimethylcyclohexanone; and wherein the phenolic compound comprises at least one of phenol, cresol, 2-isopropylphenol, and 2-phenylphenol.

Embodiment 4: The method of any one of the preceding embodiments, wherein the phenolic compound comprises phenol; the oxomethylene compound comprises acetone; and the bisphenol comprises bisphenol A. The phenolic compound can consist essentially of phenol and the oxomethylene compound can consist essentially of acetone.

Embodiment 5: The method of any one of the preceding embodiments, wherein the feeding the dehydrating agent comprises feeding the dehydrating agent in a gas phase into a bottom end of the reactive separation column and wherein the dehydrating agent comprises at least one of acetone and an inert gas, preferably, nitrogen.

Embodiment 6: The method of any one of the preceding embodiments, wherein the feeding the dehydrating agent comprises feeding the dehydrating agent in a liquid phase into the reactive separation column and wherein the dehydrating agent is the oxomethylene compound and a total amount of the oxomethylene compound added to the reactive separation column is greater than or equal to 70 wt%, or 75 to 90 wt% based on a total weight of the phenolic compound and the oxomethylene compound fed into the reactive separation column.

Embodiment 7: The method of any one of the preceding embodiments, wherein the reactive separation column operates under isothermal reaction conditions at a temperature of 55 to 70°C, or 60 to 70°C.

Embodiment 8: The method of any one of the preceding embodiments, wherein the method comprises one or both of feeding the oxomethylene compound and/or the phenolic compound at more than one feed locations, or 2 to 10 feed locations along a height of the reactive separation column and feeding the dehydrating agent at more than one dehydrating feed locations, or 2 to 10 dehydrating feed locations along the height of the reactive separation column.

Embodiment 9: The method of any one of the preceding embodiments, wherein a residence time in the reactive separation column is less than or equal to 10 minutes, or 3 to 10 minutes, or 5 to 6 minutes.

Embodiment 10: The method of any one of the preceding embodiments, wherein the method is a single pass method comprising one or both of adding 0 wt% of the bisphenol back into the column.

Embodiment 11: The method of any one of the preceding embodiments, wherein the catalyst comprises catalyst particles located on a packing support; wherein the packing support has a void space of greater than or equal to 50 vol% based on a total volume of the packing support.

Embodiment 12: The method of Embodiment 11, wherein the packing support comprises at least one of a metal foam, a wire mesh, and a corrugated metal sheet having a plurality of through-holes.

Embodiment 13: The method of any one of the preceding embodiments, further comprising separating the bisphenol stream into a separated bisphenol stream comprising the bisphenol and an unreacted recycle stream comprising an unreacted phenolic compound and an unreacted oxomethylene compound in a separation column, and optionally adding the unreacted phenolic compound and the unreacted oxomethylene compound back into the reactive separation column.

Embodiment 14: The method of Embodiment 13, wherein the bisphenol comprises para,para-bisphenol A and ortho,para-bisphenol A; and the para,para-bisphenol A is present in an amount of greater than or equal to 80 wt%, or greater than or equal to 90 wt% in the bisphenol stream based on a total weight of the para,para-bisphenol A and the ortho,para-bisphenol A.

Embodiment 15: The method of any one of the preceding embodiments, further comprising recovering the dehydrating agent from the water stream and optionally directing the dehydrating agent back into the reactive separation column.

Embodiment 16: The method of any one of the preceding embodiments, wherein the phenolic compound is present in an amount of 5 to 94 wt%, or 10 to 90 wt%, or 20 to 20 wt%, or 50 to 70 wt% based on a total weight of the phenolic compound and the oxomethylene compound fed into the reactive separation column. The phenolic compound can be present in an amount of 5 to 94 wt%, or 10 to 93 wt% based on a total weight of the phenolic compound and the oxomethylene compound fed into the reactive separation column.

Embodiment 17: The method of any one of the preceding embodiments, further comprising adding a bulk promoter to the reactive separation column.

Embodiment 18: The method of any one of the preceding embodiments, wherein the feed to the reactive separation column comprises one or both of less than or equal to 2 wt%, or 0 to 2 wt%, or 0 to 1 wt% of water based on the total weight of the feed and methanol in an amount of 250 to 5,000 ppm, or 250 to 4,000 ppm, or 260 to 3,000 ppm, or 260 to 2,000 ppm based on the total weight of the oxomethylene compound.

Embodiment 19: The method of any one of the preceding embodiments, wherein the dehydrating agent comprises a gas phase agent and the method comprises adding the gas phase dehydrating agent to the reactive separation column at a velocity of 0.01 to 0.6 m/s, or 0.05 to 0.45 m/s.

Embodiment 20: The method of any one of the preceding embodiments, wherein the dehydrating agent comprises an inert gas and the method comprises adding the inert gas at a temperature of 45 to 80°C, or 55 to 85°C.

Embodiment 21: The method of any one of the preceding embodiments, wherein the dehydrating agent comprises a gas phase agent and the method comprising adding the dehydrating agent to the reactive separation column via one or more of a side stream and a bottom stream.

Embodiment 22: The method of any one of the preceding embodiments, wherein the dehydrating agent comprises a liquid phase agent and the method comprising adding the dehydrating agent to the reactive separation column via one or more of a top stream, a side stream, and a bottom stream.

Embodiment 23: The method of any one of the preceding embodiments, wherein the reactive separation column operates at a residence time of 2 to 90 min, or 2 to 60 min, or less than or equal to 10 minutes, or 3 to 10 minutes, or 5 to 6 minutes.

Embodiment 24: The method of any one of the preceding embodiments, wherein the catalyst comprise an ion-exchange resin and optionally one or both of an attached promotor and a bulk promoter.

Embodiment 24: The method of any one of the preceding embodiments, wherein the selectivity for p,p-Bisphenol A is greater than or equal to 90%, or 90 to 99%.

The compositions, methods, and articles can alternatively comprise, consist of, or consist essentially of, any appropriate materials, steps, or components herein disclosed. The compositions, methods, and articles can additionally, or alternatively, be formulated so as to be devoid, or substantially free, of any materials (or species), steps, or components, that are otherwise not necessary to the achievement of the function or objectives of the compositions, methods, and articles.

The terms "a" and "an" do not denote a limitation of quantity, but rather denote the presence of at least one of the referenced item. The term "or" means "and/or" unless clearly indicated otherwise by context. Reference throughout the specification to "an embodiment", "another embodiment", "some embodiments", and so forth, means that a particular element (e.g., feature, structure, step, or characteristic) described in connection with the embodiment is included in at least one embodiment described herein, and may or may not be present in other embodiments. In addition, it is to be understood that the described elements may be combined in any suitable manner in the various embodiments.

In general, the compositions, methods, and articles can alternatively comprise, consist of, or consist essentially of, any ingredients, steps, or components herein disclosed. The compositions, methods, and articles can additionally, or alternatively, be formulated, conducted, or manufactured so as to be devoid, or substantially free, of any ingredients, steps, or components not necessary to the achievement of the function or objectives of the present claims.

"Optional" or "optionally" means that the subsequently described event or circumstance may or may not occur, and that the description includes instances where the event occurs and instances where it does not.

Unless specified to the contrary herein, all test standards are the most recent standard in effect as of the filing date of this application, or, if priority is claimed, the filing date of the earliest priority application in which the test standard appears.

The endpoints of all ranges directed to the same component or property are inclusive of the endpoints, are independently combinable, and include all intermediate points and ranges. For example, ranges of "up to 25 wt%, or 5 to 20 wt%" is inclusive of the endpoints and all intermediate values of the ranges of "5 to 25 wt%," such as 10 to 23 wt%, etc. Unless defined otherwise, technical and scientific terms used herein have the same meaning as is commonly understood by one of skill in the art to which this invention belongs.

All cited patents, patent applications, and other references are incorporated herein by reference in their entirety. However, if a term in the present application contradicts or conflicts with a term in the incorporated reference, the term from the present application takes precedence over the conflicting term from the incorporated reference.

While particular embodiments have been described, alternatives, modifications, variations, improvements, and substantial equivalents that are or may be presently unforeseen may arise to applicants or others skilled in the art. Accordingly, the appended claims as filed and as they may be amended are intended to embrace all such alternatives, modifications variations, improvements, and substantial equivalents.

## Claims

1. A method of forming a bisphenol, comprising:
feeding a phenolic compound and an oxomethylene compound into a reactive separation column comprising a catalyst and reacting the phenolic compound and the oxomethylene compound to produce the bisphenol and water; wherein the feeding comprises feeding the oxomethylene compound in the reactive separation column in an amount of greater than or equal to 6 wt% based on a total weight of the phenolic compound and the oxomethylene compound; wherein the oxomethylene compound has a formula: R^{k1}-(C=O)-R^{k2}, wherein R^{k1} and R^{k2} are each independently hydrogen, a halide, or a substituted or unsubstituted C₁₋₁₈ aliphatic group, wherein R^{k1} and R^{k2} can together form a C₅-₁₈ cycloalkyl group or a C₅-₁₈ cycloalkylene group;
feeding a dehydrating agent into the reactive separation column and removing a water stream comprising the water and the dehydrating agent from a top end of the reactive separation column; and
removing a bisphenol stream comprising the bisphenol from the reactive separation column.

2. The method of Claim 1, wherein the oxomethylene compound is present in an amount of 6 to 95%, or 10 to 90 wt%, or 20 to 80 wt%, or 30 to 50 wt% based on a total weight of the phenolic compound and the oxomethylene compound fed into the reactive separation column.

3. The method of any one of the preceding claims, wherein the oxomethylene compound comprises at least one of acetone, acetophenone, hexafluoroacetone, butanone, benzophenone, acetaldehyde, formaldehyde, substituted or unsubstituted cyclohexanone, and 3,3,5- trimethylcyclohexanone; and wherein the phenolic compound comprises at least one of phenol, cresol, 2-isopropylphenol, and 2-phenylphenol.

4. The method of any one of the preceding claims, wherein the phenolic compound comprises phenol; the oxomethylene compound comprises acetone; and the bisphenol comprises bisphenol A.

5. The method of any one of the preceding claims, wherein the feeding the dehydrating agent comprises feeding the dehydrating agent in a gas phase into a bottom end of the reactive separation column and wherein the dehydrating agent comprises at least one of acetone and an inert gas, preferably, nitrogen.

6. The method of any one of the preceding claims, wherein the feeding the dehydrating agent comprises feeding the dehydrating agent in a liquid phase into the reactive separation column and wherein the dehydrating agent is the oxomethylene compound and a total amount of the oxomethylene compound added to the reactive separation column is greater than or equal to 70 wt%, or 75 to 90 wt% based on a total weight of the phenolic compound and the oxomethylene compound fed into the reactive separation column.

7. The method of any one of the preceding claims, wherein the reactive separation column operates under isothermal reaction conditions at a temperature of 55 to 70°C, or 60 to 70°C.

8. The method of any one of the preceding claims, wherein the method comprises one or both of feeding the oxomethylene compound and/or the phenolic compound at more than one feed locations, or 2 to 10 feed locations along a height of the reactive separation column and feeding the dehydrating agent at more than one dehydrating feed locations, or 2 to 10 dehydrating feed locations along the height of the reactive separation column.

9. The method of any one of the preceding claims, wherein a residence time in the reactive separation column is less than or equal to 10 minutes, or 3 to 10 minutes, or 5 to 6 minutes.

10. The method of any one of the preceding claims, wherein the method is a single pass method comprising one or both of adding 0 wt% of the bisphenol back into the column.

11. The method of any one of the preceding claims, wherein the catalyst comprises catalyst particles located on a packing support; wherein the packing support has a void space of greater than or equal to 50 vol% based on a total volume of the packing support.

12. The method of Claim 11, wherein the packing support comprises at least one of a metal foam, a wire mesh, and a corrugated metal sheet having a plurality of through-holes.

13. The method of any one of the preceding claims, further comprising separating the bisphenol stream into a separated bisphenol stream comprising the bisphenol and an unreacted recycle stream comprising an unreacted phenolic compound and an unreacted oxomethylene compound in a separation column, and optionally adding the unreacted phenolic compound and the unreacted oxomethylene compound back into the reactive separation column.

14. The method of Claim 13, wherein the bisphenol comprises para,para-bisphenol A and ortho,para-bisphenol A; and the para,para-bisphenol A is present in an amount of greater than or equal to 80 wt%, or greater than or equal to 90 wt% in the bisphenol stream based on a total weight of the para,para-bisphenol A and the ortho,para-bisphenol A.

15. The method of any one of the preceding claims, further comprising recovering the dehydrating agent from the water stream and optionally directing the dehydrating agent back into the reactive separation column.
